# EUROPEAN PATENT APPLICATION

(11) **EP 4 621 386 A1**
(43) Date of publication of application: **24.09.2025**
(21) Application number: 25158222.7
(22) Date of filing: 17.02.2025
(51) Int. Cl.: G01N 21/33, G01J 1/42, G01N 27/66, G01N 33/00

(54) **PHOTOIONIZATION DETECTOR (PID) FOR DETECTING GAS**

(30) Priority: 22.03.2024 CN 202410341428
(71) Applicant: Life Safety Distribution GmbH, 1180 Rolle (CH)
(72) Inventor: HUANG, Chuang, Charlotte, 28202 (US); LIANG, Feng, Charlotte, 28202 (US); ZHU, Hao, Charlotte, 28202 (US); HU, Yu, Charlotte, 28202 (US)
(74) Representative: Haseltine Lake Kempner LLP

(57) **Abstract**

A photoionization detector (PID) is disclosed. The PID comprises a primary pole and a secondary pole spaced apart from the primary pole. The secondary pole is coupled to a bias voltage source. The secondary pole is exposed to ultraviolet (UV) light emitted from at least one UV light source for generating photo-induced electrons. The PID further comprises a at least one gas port associated with the secondary pole configured to flow gas in between the primary pole and the secondary pole for absorbing the UV light to alter the generated photo-induced electrons.

## Description

### TECHNOLOGICAL FIELD

Example embodiments of the present disclosure relate generally to gas sensors, and more particularly, to a photoionization detector (PID) for detecting high ionization energy gas.

### BACKGROUND

A photoionization detector (PID) is a type of gas detector that measures volatile organic compounds and other gases using ultraviolet (UV) light. Conventional PIDs can only detect limited types of gases because of limited capabilities of UV photons to ionize certain gas molecules.

The inventors have identified numerous areas of improvement in the existing technologies and processes, which are the subjects of embodiments described herein. Through applied effort, ingenuity, and innovation, many of these deficiencies, challenges, and problems have been solved by developing solutions that are included in embodiments of the present disclosure, some examples of which are described in detail herein.

### BRIEF SUMMARY

The following presents a summary of some example embodiments to provide a basic understanding of some aspects of the present disclosure. This summary is not an extensive overview and is intended to neither identify key or critical elements nor delineate the scope of such elements. It will also be appreciated that the scope of the disclosure encompasses many potential embodiments in addition to those here summarized, some of which will be further described in the detailed description that is presented later.

In an example embodiment, a photoionization detector (PID) is disclosed. The PID comprises a primary pole and a secondary pole spaced apart from the primary pole. The secondary pole is coupled to a bias voltage source. The secondary pole is exposed to ultraviolet (UV) light emitted from at least one UV light source for generating photo-induced electrons. The PID further comprises a at least one gas port associated with the secondary pole configured to flow gas in between the primary pole and the secondary pole for absorbing the UV light to alter the generated photo-induced electrons.

In some embodiments, the secondary pole is exposed to the UV light through one or more openings in the primary pole. In some embodiments, the photo-induced electrons are configured to generate current based at least on a voltage difference between the primary pole and the secondary pole. Further, at least one signal processing circuit is operationally coupled with the at least one secondary pole, wherein the at least one signal processing circuit is configured to receive the current. Further, the at least one signal processing circuit is configured to generate a signal related to a concentration of gas based on the received current.

In some embodiments, the primary pole and the secondary pole are insulated with a plurality of panels disposed horizontally with respect to the primary pole and the secondary pole. In some embodiments, the plurality of panels comprises a first panel disposed below the primary pole, a second panel disposed horizontally between the primary pole and the secondary pole, and a third panel disposed above the secondary pole. Further, the at least one gas port is associated with the second panel and the third panel.

In some embodiments, the gas corresponds to a high ionization energy gas. In some embodiments, the at least one UV light source is connected with a high voltage source, and corresponds to a UV lamp. Further, the UV light comprises a plurality of photons that are absorbed by the gas to alter the generated photo-induced electrons.

In another example embodiment, a method for photoionization detector (PID) is disclosed. The method comprises steps of exposing, via at least one ultraviolet (UV) light source, a secondary pole to UV light to generate photo-induced electrons, wherein the secondary pole is spaced apart from a primary pole and coupled to a bias voltage source. Further, the method comprises the steps of facilitating, via at least one gas port associated with the secondary pole, flow of gas in between the primary pole and the secondary pole, for absorbing the UV light to alter the generated photo-induced electrons.

The above summary is provided merely for purposes of summarizing some example embodiments to provide a basic understanding of some aspects of the present disclosure. Accordingly, it will be appreciated that the above-described embodiments are merely examples and should not be construed to narrow the scope or spirit of the present disclosure in any way. It will be appreciated that the scope of the present disclosure encompasses many potential embodiments in addition to those here summarized, some of which will be further described below.

### BRIEF DESCRIPTION OF THE DRAWINGS

Having thus described certain example embodiments of the present disclosure in general terms, reference will hereinafter be made to the accompanying drawings, which are not necessarily drawn to scale, and wherein:
FIG. 1 illustrates a schematic view of a photoionization detector (PID) in accordance with an example embodiment of the present disclosure;
FIG. 2 illustrates an exploded view of the PID in accordance with an example embodiment of the present disclosure;
FIG. 3 illustrates a schematic view of another photoionization detector (PID) in accordance with an example embodiment of the present disclosure;
FIG. 4 illustrates an exploded view of the another PID in accordance with an example embodiment of the present disclosure;
FIG. 5 illustrates a circuit diagram showing the PID of FIG. 1. in communication with at least one signal processing circuit in accordance with an example embodiment of the present disclosure;
FIG. 6 illustrates a block diagram of a device comprising the PID in accordance with an example embodiment of the present disclosure; and
FIG. 7 illustrates a flowchart of a method for PID in accordance with an example embodiment of the present disclosure.

### DETAILED DESCRIPTION

Some embodiments will now be described more fully hereinafter with reference to the accompanying drawings, in which some, but not all, embodiments of the present disclosure are shown. Indeed, various embodiments may be embodied in many different forms and should not be construed as limited to the embodiments set forth herein; rather, these embodiments are provided so that this disclosure will satisfy applicable legal requirements.

The components illustrated in the figures represent components that may or may not be present in various embodiments of the present disclosure described herein such that embodiments may include fewer or more components than those shown in the figures while not departing from the scope of the present disclosure. Some components may be omitted from one or more figures or shown in dashed line for visibility of the underlying components.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in various embodiments," "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "example" or "exemplary" is used herein to mean "serving as an example, instance, or illustration." Any implementation described herein as "exemplary" is not necessarily to be construed as preferred or advantageous over other implementations.

If the specification states a component or feature "may," "can," "could," "should," "would," "preferably," "possibly," "typically," "optionally," "for example," "often," or "might" (or other such language) be included or have a characteristic, that a specific component or feature is not required to be included or to have the characteristic. Such a component or feature may be optionally included in some embodiments or it may be excluded.

The present disclosure provides various embodiments of a photoionization detector (PID). Embodiments may generate photo-induced electrons by exposing a secondary pole to ultraviolet (UV) light. Embodiments may facilitate flow of gas in between a primary pole and the secondary pole for absorbing the UV light, to alter the generated photo-induced electrons. Embodiments may be configured to generate current based at least on a voltage difference between the primary pole and the secondary pole. Embodiments of the present disclosure may be configured to generate a signal related to a concentration of gas based on the received current.

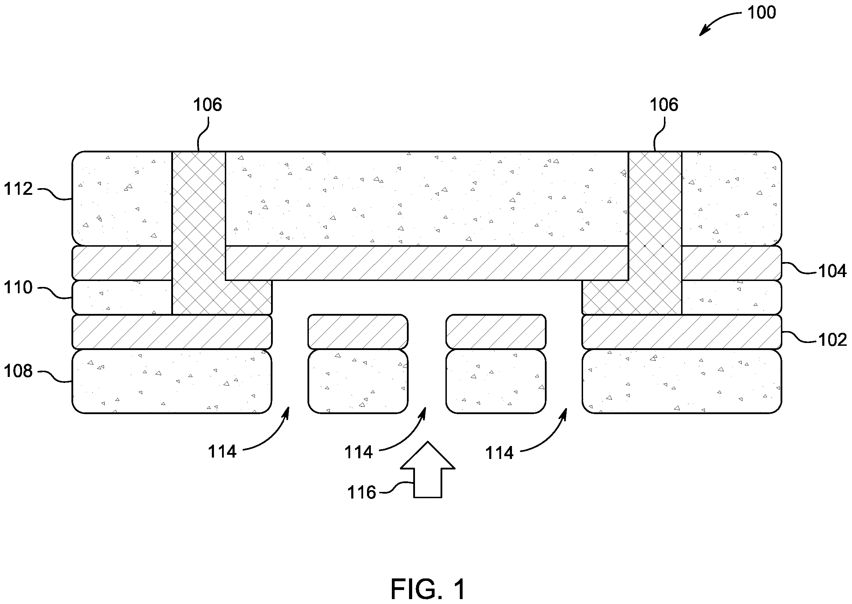

FIG. 1 illustrates a schematic view of a photoionization detector (PID) 100 in accordance with an example embodiment of the present disclosure. The PID 100 may comprise a primary pole 102, a secondary pole 104, and at least one gas port 106.

In some embodiments, the secondary pole 104 may be spaced apart from the primary pole 102. The secondary pole 104 may be coupled to a bias voltage source (not shown). In one example embodiment, the primary pole 102 and the secondary pole 104 may correspond to a positively charged anode and a negatively charged cathode, respectively. In another example embodiment, the primary pole 102 and the secondary pole 104 may correspond to a negatively charged cathode and a positively charged anode, respectively.

In some embodiments, the primary pole 102 and the secondary pole 104 may be insulated with a plurality of panels. The plurality of panels may be disposed horizontally with respect to the primary pole 102 and the secondary pole 104. The plurality of panels may be configured to provide insulation, thermal stability, and protection for the primary pole 102 and the secondary pole 104. The plurality of panels may further comprise a first panel 108, a second panel 110, and a third panel 112. The first panel 108 may be disposed below the primary pole 102. The primary pole 102 may be attached with the first panel 108. The second panel 110 may be disposed horizontally between the primary pole 102 and the secondary pole 104. In some embodiments, the second panel 110 may separate the primary pole 102 and the secondary pole 104.The third panel 112 may be disposed above the secondary pole 104.

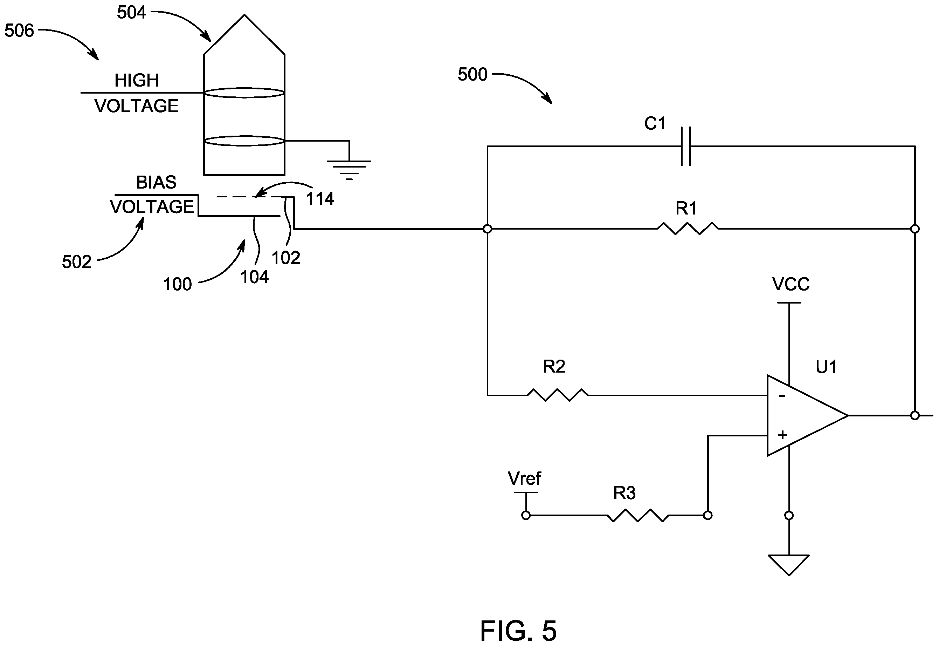

In some embodiments, the secondary pole 104 may be exposed to an ultraviolet (UV) light using an at least one UV light source (not shown). The description related to the UV light source will be described in conjunction with FIG. 5. It will be apparent to one skilled in the art that the UV light comprises a plurality of photons. The UV light may be emitted from at least one UV light source (not shown). The secondary pole 104 may be exposed to the UV light through one or more openings 114 in the primary pole 102 and the first panel 108, as indicated by an arrow 116. Further, the one or more openings 114 may allow the UV light to reach the secondary pole 104 and a space between the primary pole 102 and the secondary pole 104. The secondary pole 104 may be exposed to the UV light comprising the plurality of photons, for generating photo-induced electrons.

In some embodiments, the PID 100 may further comprise the at least one gas port 106. The at least one gas port 106 may be associated with the secondary pole 104. In some embodiments, the at least one gas port 106 may be associated with the second panel 110 and the third panel 112. Further, the at least one gas port 106 may be configured to facilitate flow of gas in between the primary pole 102 and the secondary pole 104 for absorbing the plurality of photons. In one example embodiment, the gas may correspond to a high ionization energy gas. Further, the gas may alter the UV light by absorbing the plurality of photons. The altered UV light may alter the photo-induced electrons.

In some embodiments, the photo-induced electrons may be configured to generate current based at least on a voltage difference between the primary pole 102 and the secondary pole 104. Further, the gas may alter the generated current by absorbing the plurality of photons altering the photo-induced electrons. In one example embodiment, alter may correspond to decrease in the generated current. In another example embodiment, alter may correspond to increase in the generated current. In some embodiments, at least one signal processing circuit (not shown) may be operationally coupled with the secondary pole 104. The at least one signal processing circuit may be configured to receive the current. Thereafter, the at least one signal processing circuit may be configured to generate a signal related to a concentration of gas based on the received current.

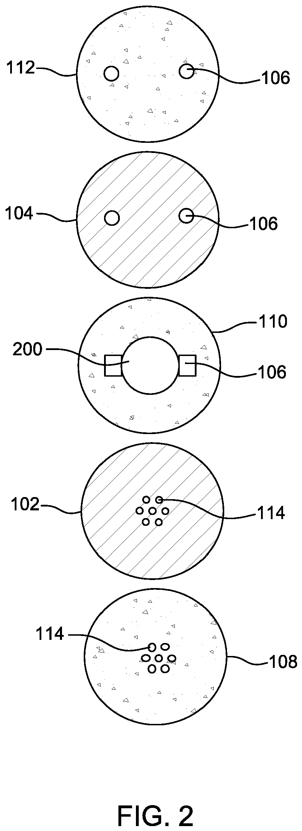

FIG. 2 illustrates an exploded view of the PID 100, in accordance with an example embodiment of the present disclosure.

As discussed herein, the secondary pole 104 may be exposed to the UV light through the one or more openings 114 in the primary pole 102 and the first panel 108. Further, the one or more openings 114 may allow the UV light to reach the secondary pole 104 and a space 200 between the primary pole 102 and the secondary pole 104. In some embodiments, the space 200 may be a cavity on the second panel 110. The first panel 108 and the primary pole 102 may comprise the one or more openings 114 to allow the secondary pole to be exposed to the UV light. In some embodiments, the one or more openings 114 may correspond to seven openings in the first panel 108 and the primary pole 102, respectively. In some embodiments, number of the one or more openings 114 may increase or decrease based on the components of the PID 100.

Further, as discussed in FIG. 1, the at least one gas port 106 may be associated with the secondary pole 104, the second panel 110, and the third panel 112. In one example embodiment, the at least one gas port in the secondary pole 104, the second panel 110, and the third panel 112 may be two gas ports. In some embodiments, the second panel 110 may comprise the space 200 between the primary pole 102 and the secondary pole 104 for generating photo-induced electrons.

It will be apparent to one skilled in the art that the number of openings and the gas port may vary, without departing from the scope of the disclosure.

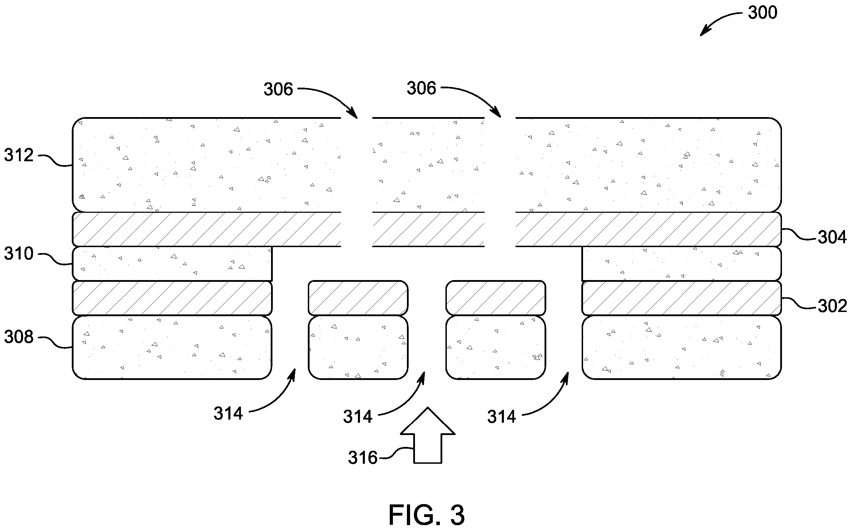

FIG. 3 illustrates a schematic view of another photoionization detector (PID) 300 in accordance with an example embodiment of the present disclosure. The PID 300 may comprise a primary pole 302, a secondary pole 304, and at least one gas port 306.

In some embodiments, the secondary pole 304 may be spaced apart from the primary pole 302. The secondary pole 304 may be coupled to a bias voltage source (not shown). In one example embodiment, the primary pole 302 and the secondary pole 304 may correspond to a positively charged anode and a negatively charged cathode, respectively. In another example embodiment, the primary pole 302 and the secondary pole 304 may correspond to a negatively charged cathode and a positively charged anode, respectively.

In some embodiments, the primary pole 302 and the secondary pole 304 may be insulated with a plurality of panels. The plurality of panels may be disposed horizontally with respect to the primary pole 302 and the secondary pole 304. The plurality of panels may be configured to provide insulation, thermal stability, and protection for the primary pole 302 and the secondary pole 304. The plurality of panels may further comprise a first panel 308, a second panel 310, and a third panel 312. The first panel 308 may be disposed below the primary pole 302. The primary pole 302 may be attached with the first panel 308. The second panel 310 may be disposed horizontally between the primary pole 302 and the secondary pole 304. In some embodiments, the second panel 310 may separate the primary pole 302 and the secondary pole 304.The third panel 312 may be disposed above the secondary pole 304.

In some embodiments, the secondary pole 304 may be exposed to an ultraviolet (UV) light using an at least one UV light source (not shown). The description related to the UV light source will be described in conjunction with FIG. 5. It will be apparent to one skilled in the art that the UV light comprises a plurality of photons. The UV light may be emitted from at least one UV light source (not shown). The secondary pole 304 may be exposed to the UV light through one or more openings 314 in the primary pole 302 and the first panel 308, as indicated by an arrow 316. Further, the one or more openings 314 may allow the UV light to reach the secondary pole 304 and a space between the primary pole 302 and the secondary pole 304. The secondary pole 304 may be exposed to the UV light comprising the plurality of photons, for generating photo-induced electrons.

In some embodiments, the PID 100 may further comprise the at least one gas port 306. The at least one gas port 306 may be associated with the secondary pole 304. In some embodiments, the at least one gas port 306 may be associated with the third panel 312. Further, the at least one gas port 306 may be configured to facilitate flow of gas in between the primary pole 302 and the secondary pole 304 for absorbing the plurality of photons. In one example embodiment, the gas may correspond to a high ionization energy gas. Further, the gas may alter the UV light by absorbing the plurality of photons. The altered UV light may alter the photo-induced electrons.

In some embodiments, the photo-induced electrons may be configured to generate current based at least on a voltage difference between the primary pole 302 and the secondary pole 304. Further, the gas may alter the generated current by absorbing the plurality of photons altering the photo-induced electrons. In one example embodiment, alter may correspond to decrease in the generated current. In another example embodiment, alter may correspond to increase in the generated current. In some embodiments, at least one signal processing circuit (not shown) may be operationally coupled with the secondary pole 304. The at least one signal processing circuit may be configured to receive the current. Thereafter, the at least one signal processing circuit may be configured to generate a signal related to a concentration of gas based on the received current.

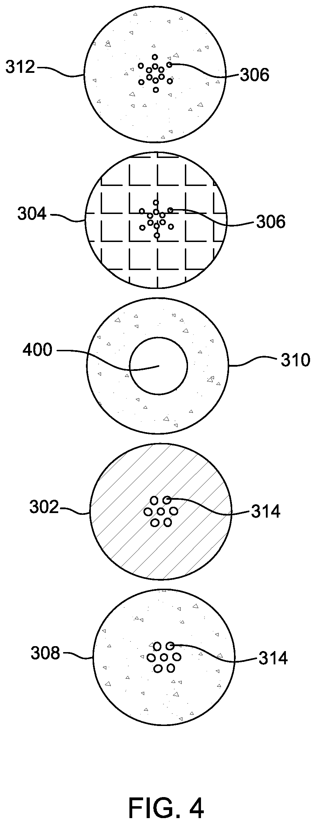

FIG. 4 illustrates an exploded view of the another PID 300, in accordance with an example embodiment of the present disclosure.

As discussed herein, the secondary pole 304 may be exposed to the UV light through one or more openings 314 in the primary pole 302 and the first panel 308. Further, the one or more openings 314 may allow the UV light to reach the secondary pole 304 and a space 400 between the primary pole 302 and the secondary pole 304. The first panel 308 and the primary pole 302 may comprise the one or more openings 314 to allow the secondary pole to be exposed to the UV light. In one example embodiment, the one or more openings 314 may correspond to seven openings in the first panel 308 and the primary pole 302, respectively. In some embodiments, number of the one or more openings 314 may increase or decrease based on the components of the PID 300.

Further, as discussed in FIG. 1, the at least one gas port 306 may be associated with the secondary pole 304, the second panel 310 and the third panel 312. In one example embodiment, the at least one gas port in the secondary pole 304, the second panel 310 and the third panel 312 may be two gas ports. In some embodiments, the second panel 310 may comprise the space 400 between the primary pole 302 and the secondary pole 304 for generating photo-induced electrons.

It will be apparent to one skilled in the art that the number of openings and the gas port may vary, without departing from the scope of the disclosure.

In some embodiments, the PID 100 and the PID 300 may hold the same functionality without departing from the scope of the disclosure.

FIG. 5 illustrates the PID 100 in communication with at least one signal processing circuit 500, in accordance with an example embodiment of the present disclosure.

As discussed in FIG. 1, the secondary pole 104 may be coupled to a bias voltage source 502. The bias voltage source 502 may facilitate an ionization process to generated photo-induced electrons and the detection of the gas. Further, the secondary pole 104 may be exposed to an ultraviolet (UV) light. The UV light may be emitted from at least one UV light source 504. Further, the at least one UV light source 504 may be connected to a high voltage source 506. In one example embodiment, the at least one UV light source 504 may correspond to a UV lamp. Further, the secondary pole 104 may be exposed to the UV light through the one or more openings 114 in the primary pole 102 and the first panel 108. Further, the one or more openings 114 may allow the UV light to reach the secondary pole 104 and the space between the primary pole 102 and the secondary pole 104 for generating photo-induced electrons.

In some embodiments, the PID 100 may further comprise the at least one gas port 106. The at least one gas port 106 may be associated with the secondary pole 104. Further, the at least one gas port 106 may be configured to flow gas in between the primary pole 102 and the secondary pole 104 for absorbing the plurality of photons. In one example embodiment, the gas may correspond to a high ionization energy gas. Further, the gas may alter the UV light by absorbing the plurality of photons. The altered UV light may alter the photo-induced electrons. In some embodiments, the at least one gas port 106 may be associated with the second panel 110 and the third panel 112.

In some embodiments, the photo-induced electrons may be configured to generate current based at least on a voltage difference between the primary pole 102 and the secondary pole 104. Further, the gas may alter the generated current by absorbing the plurality of photons altering the photo-induced electrons. In some embodiments, the at least one signal processing circuit 500 may be configured to receive the altered current. Further, the at least one signal processing circuit 500 may be configured to generate a signal related to a concentration of gas based on the received altered current. Further, the generated signal may be configured to send a notification on one or more user devices (not shown) to alert the one or more users about the concentration of the gas.

In some example embodiments, the concentration of the gas may be determined based at least on a formula. In one example, assume I1 is the photo induced current caused by UV light and the strength of the I1 is S1. When there is high ionization energy gas input, part of the UV light will be absorbed. Further, assume P1 is the absorption rate, which is a function of gas concentration-Cgas. At a simple case, P1=K2*Cgas1. K2 is the index. The index may be different for different gases. Then, based on the formula I1 =k1*S1(1-P1)= k1*S1(1-k2*Cgas1), the concentration of the gas may be determined as Cgas1=(1-I1/k1*S1)/k2.

In some embodiments, the at least one signal processing circuit 500 may comprise aa plurality of components to generate the signal. The plurality of components may include a capacitor C1, a resistor R1, a resistor R2, a resistor R3, and a comparator U1 to generate the signal based on the received current. The signal may be related to the concentration of gas based on the received current.

In some alternate embodiments, the PID 300 may be coupled with the at least one signal processing circuit 500 and the at least one UV light source 504. It will be apparent to one skilled in the art the above-mentioned components of the PID 100 and PID 300 have been provided only for illustration purposes, without departing from the scope of the disclosure.

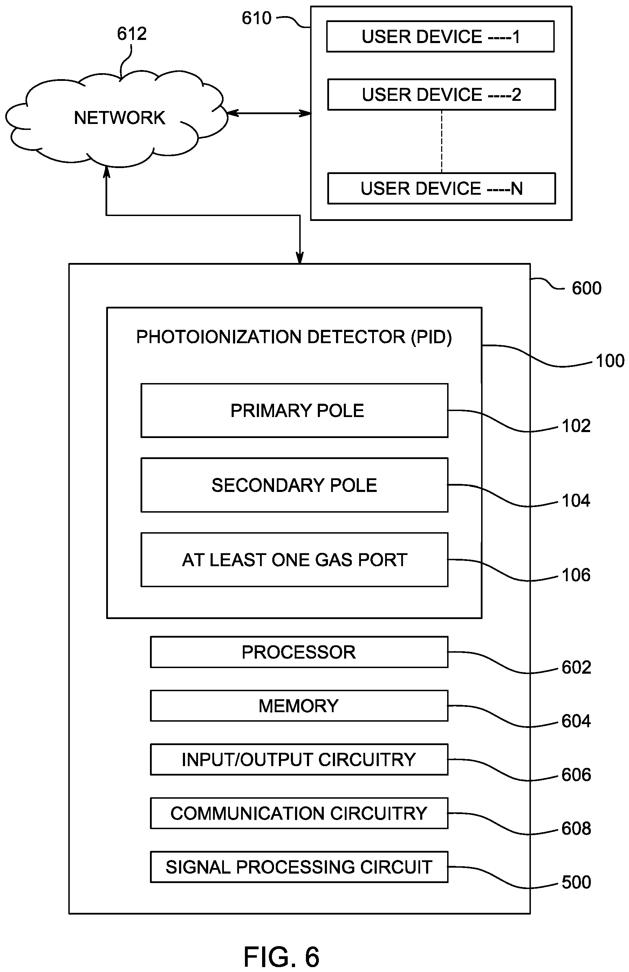

FIG. 6 illustrates a block diagram of a device 600 comprising the PID 100, in accordance with an example embodiment of the present disclosure. The device 600 may comprise the PID 100, at least one processor 602, a memory 604, an input/ output circuitry 606, a communication circuitry 608, and the at least one signal processing circuit 500.

In some embodiments, the PID 100 may comprise the primary pole 102 and the secondary pole 104 spaced apart from the primary pole 102. The secondary pole 104 may be exposed to UV light emitted from the at least one UV light source 504 for generating photo-induced electrons. The PID 100 further comprises the at least one gas port 106 associated with the secondary pole 104 configured to flow gas in between the primary pole 102 and the secondary pole 104 for absorbing the plurality of photons. In one example embodiment, the gas may correspond to a high ionization energy gas. Further, the gas may alter the UV light by absorbing the plurality of photons. The altered UV light may alter the photo-induced electrons.

In some embodiments, the photo-induced electrons may be configured to generate current based at least on a voltage difference between the primary pole 102 and the secondary pole 104. Further, the gas may alter the generated current by absorbing the plurality of photons altering the photo-induced electrons. In some embodiments, the at least one signal processing circuit 500 may be configured to receive the altered current. Further, the at least one signal processing circuit 500 may be configured to generate a signal related to the concentration of gas based on the received altered current. Further, the generated signal may be configured to send a notification on the one or more user devices 610, comprising the concentration of the gas.

Further, the device 600 may comprise at least one processor 602. The at least one processor 602 may be configured to receive a signal related to the concentration of gas based on the received altered current. The at least one processor 602 may be configured to analyze the received signal to determine the concentration of the gas. In some embodiments, the at least one processor 602 may include suitable logic, circuitry, and/or interfaces that are operable to execute one or more instructions stored in the memory 604 to perform predetermined operations. In one embodiment, the at least one processor 602 may be configured to decode and execute any instructions received from one or more other electronic devices or server(s). The at least one processor 602 may be configured to execute one or more computer-readable program instructions, such as program instructions to carry out any of the functions described in this description. Further, the processor may be implemented using one or more processor technologies known in the art. Examples of the processor include, but are not limited to, one or more general purpose processors (e.g., INTEL^{®} or Advanced Micro Devices^{®} (AMD) microprocessors) and/or one or more special purpose processors (e.g., digital signal processors or Xilinx^{®} System On Chip (SOC) Field Programmable Gate Array (FPGA) processor).

Further, the memory 604 may be communicatively coupled to the at least one processor 602. Further, the memory 604 may be configured to store a set of instructions and data executed by the at least one processor 602. Further, the memory 604 may include the one or more instructions that are executable by the at least one processor 602 to perform specific operations. In some embodiments, the memory 604 may be configured to include one or more instructions to receive a signal related to the concentration of gas based on the received altered current. Further, the memory 604 may be configured to include one or more instructions to analyze the received signal to determine the concentration of the gas. It is apparent to a skilled artisan that the one or more instructions stored in the memory 604 enable the device 600 to perform the predetermined operations. Some of the commonly known memory implementations include, but are not limited to, fixed (hard) drives, magnetic tape, floppy diskettes, optical disks, Compact Disc Read-Only Memories (CD-ROMs), and magneto-optical disks, semiconductor memories, such as ROMs, Random Access Memories (RAMs), Programmable Read-Only Memories (PROMs), Erasable PROMs (EPROMs), Electrically Erasable PROMs (EEPROMs), flash memory, magnetic or optical cards, or other type of media/machine-readable medium suitable for storing electronic instructions.

In some embodiments, the device 600 may further comprise the input/output circuity 606. The input/output circuitry 606 may enable a user to communicate or interface with the PID 100, via one or more user devices 610. The one or more user devices 610 may include N number of user devices. In some embodiments, the input/output circuitry 606 may act as a medium to transmit input from the interface to and from the device 600. In some embodiments, the input/output circuitry 606 may refer to the hardware and software components that facilitate the exchange of information between one or more user devices 610 and the device 600. In one example, the device 600 may include a graphical user interface (GUI) (not shown) as input circuitry to allow the one or more users to input data. The input/output circuitry 606 may include various input devices such as keyboards, barcode scanners, GUI for the one or more users to provide data and various output devices such as displays, printers for the one or more users to receive data. In another example, the input/output circuitry 606 may include various output circuitry such as a display to indicate the concentration of the gas.

In some embodiments, the device 600 may further comprise the communication circuitry 608. The communication circuitry 608 may allow the device 600 and the PID 100 to exchange data or information with other systems or apparatuses. Further, the communication circuitry 608 may include network interfaces, protocols, and software modules responsible for sending and receiving data or information. In some embodiments, the communication circuitry 608 may include Ethernet ports, Wi-Fi adapters, or communication protocols like HTTP or MQTT for connecting with other systems. The communication circuitry 608 may allow the device 600 to stay up-to-date and accurately track the concentration of the gas.

In some embodiments, the device 600 may be communicatively coupled to the one or more user devices 610, via a network 612. The network 612 may facilitate a communication link between the device 600 and the one or more user devices 610. In some embodiments, the network 612 may further facilitate a communication link among the other components of the device 600. Further, the network 612 may be a wireless network and/or a wired network. The network 612 may be implemented using one or more communication techniques. The one or more communication techniques may be Radio waves, Wi-Fi, Bluetooth, ZigBee, Z-wave and other communication techniques, known in the art.

In some embodiments, the one or more user devices 610 may allow one or more users to send one or more commands i.e., input commands to the device 600 to determine the concentration of the gas. The one or more input commands may include, but is not limited to, checking the concentration, decreasing the flow of gas, or executing a "script" of commands with varying time intervals to determine concentration at the time intervals. In some embodiments, the one or more user devices 610 may include a smartphone, a tablet, a laptop, a personal computer (PC), or a smart watch.

In some embodiments, the device 600 may correspond to at least one of a portable gas detector, indoor air quality (IAQ) monitor, gas chromatograph or any other device known in the art that uses the PID 100 to determine the concentration of the gas. It will be apparent to one skilled in the art the above-mentioned components of the device 600 have been provided only for illustration purposes, without departing from the scope of the disclosure.

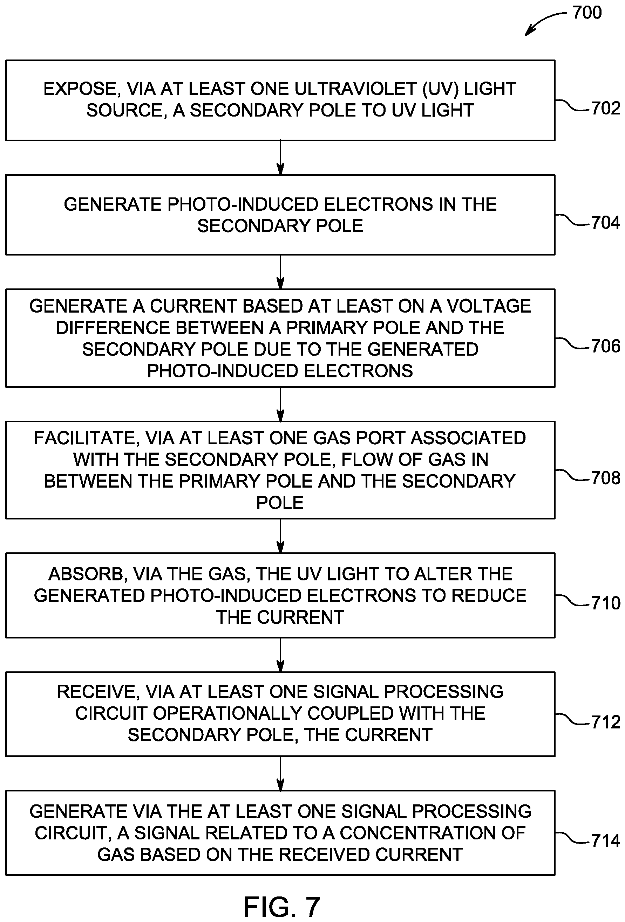

FIG. 7 illustrates a flowchart of a method 700 for PID 100, in accordance with an example embodiment of the present disclosure.

At operation 702, the secondary pole 104 is exposed to UV light, via the at least one UV light source 504. The secondary pole 104 may be spaced apart from the primary pole 102. The secondary pole 104 may be exposed to the UV light through the one or more openings 114 in the primary pole 102. In some embodiments, the primary pole 102 and the secondary pole 104 may be fixed and insulated with a plurality of panels. The plurality of panels may be disposed horizontally with respect to the primary pole 102 and the secondary pole 104. The plurality of panels may be configured to provide insulation, thermal stability, and protection for the primary pole 102 and the secondary pole 104.

In some embodiments, the plurality of panels may further comprise the first panel 108, the second panel 110, and the third panel 112. The first panel 108 may be disposed below the primary pole 102. The primary pole 102 may be attached with the first panel 108. The second panel 110 may be disposed horizontally between the primary pole 102 and the secondary pole 104. The second panel 110 may separate the primary pole 102 and the secondary pole 104. The third panel 112 may be disposed above the secondary pole 104.

At operation 704, photo-induced electrons are generated in the secondary pole 104. The secondary pole 104 may be exposed to the UV light comprising the plurality of photons, for generating photo-induced electrons. The photo-induced electrons may generate a voltage difference between the primary pole 102 and the secondary pole 104. At operation 706, the current is generated based at least on the voltage difference between the primary pole 102 and the secondary pole 104 due to the generated photo-induced electrons. At operation 708, flow of gas is facilitated in between the primary pole 102 and the secondary pole 104, via the at least one gas port 106 associated with the secondary pole 104. In some embodiments, the gas may correspond to a high ionization energy gas. For example, carbonyl sulfide (COS) gas is facilitated in between the primary pole 102 and the secondary pole 104.

At operation 710, the UV light is absorbed by the gas to alter the generated photo-induced electrons to reduce the current. In some embodiments, the gas may alter the UV light by absorbing the plurality of photons of the UV light. In one example embodiment, alter may correspond to decrease the photo-induced electrons. In another example embodiment, alter may correspond to increase the photo-induced electrons. For example, the COS gas absorbs the plurality of photons of the UV light to decrease the generated photo-induced electrons to reduce the current. At operation 712, the current is received, via the at least one signal processing circuit 500 operationally coupled with the secondary pole 104. For example, the at least one signal processing circuit 500 receives the reduced current. At operation 714, the signal is generated, via the at least one signal processing circuit 500, related to the concentration of gas based on the received current. In some embodiments, the signal may be related to the concentration of gas based on the received current. For example, the at least one signal processing circuit 500 generate a signal related to the concentration of the COS gas based on the received reduced current.

The present disclosure involves utilizing the PID to determine the concentration of the gas. The PID is exposed to UV light, to generate photo-induced electron for high ionization energy gas detection. The PID integrate photo-induced electron generator with bias voltage. The PID provides quick response time in applications where fast detection of hazardous substances is essential, such as in emergency response situations. The PID allows for the detection of low concentrations of gases, making the PID valuable in applications where even trace amounts of certain substances are of concern. The PID allows for ease of use in field applications, environmental monitoring, industrial hygiene, and emergency response.

Many modifications and other embodiments of the disclosure set forth herein will come to mind to one skilled in the art to which the present disclosure pertains having the benefit of the teachings presented in the foregoing descriptions and the associated drawings. Therefore, it is to be understood that the present disclosure is not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A photoionization detector (PID) comprising:
a primary pole;
a secondary pole spaced apart from the primary pole and coupled to a bias voltage source, wherein the secondary pole is exposed to ultraviolet (UV) light emitted from at least one UV light source for generating photo-induced electrons; and
at least one gas port associated with the secondary pole configured to flow gas in between the primary pole and the secondary pole for absorbing the UV light to alter the generated photo-induced electrons.

2. The photoionization detector (PID) of claim 1, wherein the secondary pole is exposed to the UV light through one or more openings in the primary pole.

3. The photoionization detector (PID) of claim 1, wherein the photo-induced electrons are configured to generate current based at least on a voltage difference between the primary pole and the secondary pole.

4. The photoionization detector (PID) of claim 3, further comprising at least one signal processing circuit is operationally coupled with the at least one secondary pole, wherein the at least one signal processing circuit is configured to receive the current.

5. The photoionization detector (PID) of claim 4, wherein the at least one signal processing circuit is configured to generate a signal related to a concentration of gas based on the received current.

6. The photoionization detector (PID) of claim 1, wherein the primary pole and the secondary pole are insulated with a plurality of panels disposed horizontally with respect to the primary pole and the secondary pole.

7. The photoionization detector (PID) of claim 6, wherein the plurality of panels comprises:
a first panel disposed below the primary pole;
a second panel disposed horizontally between the primary pole and the secondary pole; and
a third panel disposed above the secondary pole.

8. The photoionization detector (PID) of claim 7, wherein the at least one gas port is associated with the second panel and the third panel.

9. The photoionization detector (PID) of claim 1, wherein the gas corresponds to a high ionization energy gas.

10. The photoionization detector (PID) of claim 1, wherein the at least one UV light source is connected with a high voltage source, and corresponds to a UV lamp, wherein the UV light comprises a plurality of photons that are absorbed by the gas to alter the generated photo-induced electrons.

11. A method for photoionization detector (PID), the method comprising:
exposing, via at least one ultraviolet (UV) light source, a secondary pole to UV light to generate photo-induced electrons, wherein the secondary pole is spaced apart from a primary pole, and coupled to a bias voltage source;
facilitating, via at least one gas port associated with the secondary pole, flow of gas in between the primary pole and the secondary pole, for absorbing the UV light to alter the generated photo-induced electrons.

12. The method of claim 11, wherein the secondary pole is exposed to the UV light through one or more openings in the primary pole.

13. The method of claim 11, wherein the photo-induced electrons are configured to generate current based at least on a voltage difference between the primary pole and the secondary pole.

14. The method of claim 13, further comprising:
receiving, via at least one signal processing circuit operationally coupled with the secondary pole, the current.

15. The method of claim 14, further comprising generating, via the at least one signal processing circuit, a signal related to a concentration of gas based on the received current.
